# EUROPÄISCHE PATENTSCHRIFT

(11) **EP 0 579 113 B1**
(45) Veröffentlichungstag und Bekanntmachung des Hinweises auf die Patenterteilung: **02.05.1997**
(21) Anmeldenummer: 93110926.8
(22) Anmeldetag: 08.07.1993
(51) Int. Cl.: C07F 9/655, C07D 319/06

(54) **Verbessertes Verfahren zur Herstellung von cyclischen Acetalen von 3-formyl-2-butenyl-triphenylphosphoniumchlorid**
Improved process for preparing cyclic acetals of 3-formyl-2-butenyl-triphenylphosphonium chloride
Procédé amélioré pour préparer des acétals cycliques du chlorure de formyl-3-butényl-2-triphénylphosphonium

(30) Priorität: 14.07.1992 DE 4223061; 31.07.1992 DE 4225322
(43) Veröffentlichungstag der Anmeldung: 19.01.1994
(73) Patentinhaber: BASF Aktiengesellschaft, 67063 Ludwigshafen (DE)
(72) Erfinder: Krause, Wolfgang, Dr., D-6800 Mannheim 81 (DE); Paust, Joachim, Dr., D-6708 Neuhofen (DE); Dobler, Walter, Dr., D-6900 Heidelberg (DE); Jaedicke, Hagen, Dr., D-6700 Ludwigshafen (DE)

(56) Entgegenhaltungen:
- JUSTUS LIEBIGS ANNALEN DER CHEMIE Nr. 12, 1976, WEINHEIM DE Seiten 2194 - 2205 JOACHIM PAUST 'Monoacetale von 2-Methyl-2-buten-1,4-dial'

## Beschreibung

Die Erfindung betrifft ein verbessertes Verfahren zur Herstellung von cyclischen Acetalen von 3-Formyl-2-butenyl-triphenylphosphoniumchlorid ausgehend von 3-Formyl-2-butenylacetat.

Bei der synthetischen Herstellung von Terpenen hat die Wittig-Reaktion große Bedeutung erlangt. So kann man mit ihrer Hilfe bei der Totalsynthese von Terpenen in wenigen Stufen zum Ziel gelangen, wenn man das Kohlenstoffgerüst aus C₅-Einheiten aufbaut. Ein Beispiel für dieses Synthesekonzept ist die Kettenverlängerung von Polyenaldehyden mit Hilfe von Acetalen von 3-Formyl-2-butenyl-triphenylphosphoniumhalogeniden. Genannt sei die Herstellung von den als Lebensmittelfarbstoffen begehrten β-Apocarotinalen durch Wittig-Olefinierung von Retinal mit Acetalen von 3-Formyl-2-butenyl-triphenylphosphoniumhalogeniden. Es hat daher nicht an Versuchen gefehlt, ein vorteilhaftes Verfahren für die Herstellung dieser Phosphoniumsalze zu finden.

So werden in Liebigs Ann. Chem. 1976, Seiten 2194 - 2205, Möglichkeiten zur Herstellung von 1,1-Dimethoxy-3-methyl-2-butenyl-triphenylphosphoniumchlorid aufgezeigt und ein Verfahren zur Herstellung der wesentlich besser geeigneten cyclischen Acetale vorgestellt. Gemäß dem hier beschriebenen Verfahren wird 3-Formyl-2-butenylacetat mit 1,3-Diolen in Toluol acetalisiert, das erhaltene Acetoxyacetal durch Umestern mit überschüssigem Methanol in das entsprechende Hydroxyacetal überführt, das Hydroxyacetal durch Vilsmeier-Chlorierung in Benzol in das entsprechende Chloracetal überführt und dieses schließlich in Toluol mit Triphenylphosphin umgesetzt. Nachteilig an diesem Verfahren ist, daß die Überführung des Verfahrens in den technischen Maßstab Schwierigkeiten bereitet und die erzielbaren Ausbeuten für eine technische Synthese unzureichend sind. So ist beispielsweise gemäß dem dort beschriebenen Verfahren ein ständiger Wechsel der für die 4 Reaktionsstufen benötigten Lösungsmittel erforderlich, was technisch sehr aufwendig ist. Zudem werden nur Ausbeuten von etwa 44 % der Theorie erzielt.

Es war daher die Aufgabe der Erfindung das oben beschriebene Verfahren so zu verbessern, daß es technisch vorteilhaft durchgeführt werden kann und zudem die erzielbaren Ausbeuten wesentlich zu verbessern.

Gegenstand der Erfindung ist daher ein verbessertes Verfahren zur Herstellung von cyclischen Acetalen des 3-Formyl-2-butenyl-triphenylphosphoniumchlorids der allgemeinen Formel I 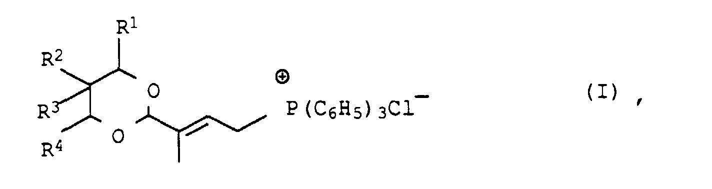 in denen
R¹, R², R³ oder R⁴ für H oder -CH₃ stehen, durch
a) Acetalisieren des 3-Formyl-butenylacetates der Formel II 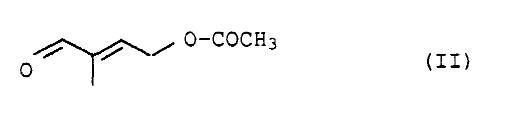 mit 1,3-Diolen der allgemeinen Formel III 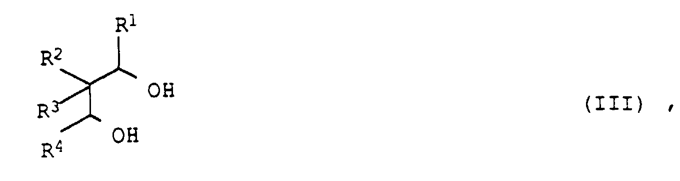 in der R¹ - R⁴ die oben angegebene Bedeutung haben,
b) Überführen der erhaltenen Acetoxyacetale der allgemeinen Formel IV in die entsprechenden Hydroxyacetale der allgemeinen Formel V 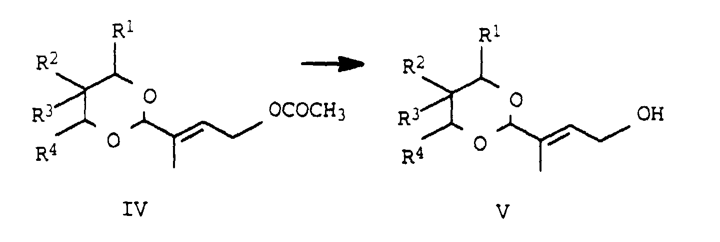
c) Vilsmeier-Chlorierung der erhaltenen Hydroxyacetale der Formel V unter Bildung der 4-Chlor-acetale der allgemeinen Formel VI 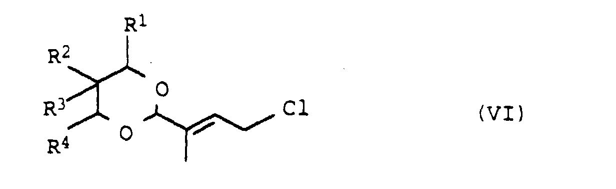 und
d) Umsetzung der 4-Chlor-acetale der Formel VI mit Triphenylphosphin,
das dadurch gekennzeichnet ist, daß man die Reaktionsschritte a) bis c) in einem aliphatischen oder cycloaliphatischen Kohlenwasserstoff oder Kohlenwasserstoffgemisch mit 6 bis 8 C-Atomen und den Reaktionsschritt d) in einem Alkanol mit 1 bis 3 C-Atomen und/oder in einem aliphatischen oder cycloaliphatischen Kohlenwasserstoff mit 6 bis 8 C-Atomen durchgeführt.

Von Vorteil ist, daß man bei dieser Verfahrensverbesserung das Überführen der Acetoxyacetale der allgemeinen Formel IV in die entsprechenden Hydroxyacetale der allgemeinen Formel V mit mindestens 5 %iger wäßriger Natronlauge durchführen kann. Besonders vorteilhaft gestaltet sich das neue Verfahren, wenn man das Überführen der Acetoxyacetale der allgemeinen Formel IV in die entsprechenden Hydroxyacetale der allgemeinen Formel V mit wäßriger Natronlauge oder Kalilauge in Gegenwart von Phasentransferkatalysatoren durchführt.

Das als Ausgangssubstanz für das erfindungsgemäße Verfahren benötigte 3-Formyl-butenylacetat der Formel II (auch β-Formylcrotylacetat genannt) ist eine bekannte Verbindung, die bei der technischen Vitamin-A-synthese eine wichtige Rolle spielt und gemäß DE-OS-20 04 675 und DE-OS-19 41 632 durch Hydroformylierung von Vinylglykolbisacetat hergestellt werden kann.

Als für die Durchführung des erfindungsgemäßen Verfahrens geeignete Kohlenwasserstoffe mit 6 bis 8 C-Atomen seien beispielsweise genannt, n-Hexan, n-Heptan, n-Octan, Cyclohexan sowie Gemische von Hexanen, Heptanen und/oder Octane, wie sie bei der Synthese anfallen. Insbesondere seien n-Heptan bzw. Heptangemische genannt.

Als Alkanole mit 1 bis 3 C-Atomen sind erfindungsgemäß Methanol, Ethanol, n-Propanol und iso-Propanol, insbesondere Methanol geeignet.
a) Die Acetalisierung des β-Formyl-crotylacetats mit dem aliphatischen 1,3-Diol der allgemeinen Formel III erfolgt erfindungsgemäß in einem der obengenannten aliphatischen oder cycloaliphatischen Kohlenwasserstoffe mit 6 bis 8 C-Atomen als Lösungsmittel. Als geeignete saure Katalysatoren für die Acetalisierung seien beispielsweise genannt para-Toluolsulfonsäure, Methansulfonsäue, Trifluormethansulfonsäure, Trichlormethansulfonsäure.
   Diese Katalysatoren werden in einer Menge von 0,01 mol-% bis 5 mol-%, bevorzugt 0,1 mol-% bis 1,0 mol-% zugesetzt. Das bei der Acetalisierung gebildete Wasser und ggf. das in der als Katalysator verwendeten Säure enthaltene Wasser werden mit Vorteil während der Umsetzung azeotrop aus dem Reaktionsgemisch entfernt. Ohne Entfernen von H₂O bleibt die Reaktion bei etwa 73 % Umsatz stehen.
   Die Reaktionstemperaturen betragen im allgemeinen 25 bis 130°C, vorzugsweise 40 bis 100°C bei Reaktionsdrücken von 1 bis 1013 mbar, vorzugsweise 100 bis 500 mbar. Die Reaktionszeiten betragen etwa 1 bis 10, vorzugsweise 2 bis 3 Stunden. Die bei der Acetalisierung gebildete organische Lösung des Acetals kann als solche in den weiteren Syntheseweg eingeführt werden.
b) Zur Überführung der Acetoxyacetale der allgemeinen Formel IV in die entsprechenden Hydroxyacetale setzt man die Acetoxyacetale erfindungsgemäß in einem der oben genannten aliphatischen oder cycloaliphatischen Kohlenwasserstoffe oder Kohlenwasserstoffgemische mit wäßriger Natronlauge oder Kalilauge um. Die Konzentration der Natronlauge sollte 3 bis 52, vorzugsweise 20 bis 50 % betragen, die der Kalilauge 3 bis 53, vorzugsweise 20 bis 50 %. Die wäßrige Alkalilauge wird mit Vorteil langsam zu dem Reaktionsgemisch zugefügt. Zur Verkürzung der Reaktionszeit, Verringerung der Alkalihydroxidmenge und Erhöhung der Ausbeute können dem Reaktionsgemisch sentransferkatalysatoren (PTK) zugefügt werden. Bezüglich näherer Angaben zu dem Begriff Phasentransfer-Katalyse verweisen wir beispielsweise auf Angew. Chem. 89 (1977) 521-33.
   Als geeignete PTK seien beispielsweise genannt Tetraalkylammoniumsalze der allgemeinen Formel R₄N^{⊕}X^{⊖} mit R=C₁-C₂₀, auch "gemischte" Alkylreste und X^{⊖}=Cl^{⊖} , Br^{⊖}, I^{⊖}; insbesondere Protectol® (BASF; Benzyl-C₁₂₋₁₄-alkyldimethylammoniumchlorid) und Aliquat® (General Mills, Inc., Minneapolis, USA). Die Reaktionstemperaturen betragen im allgemeinen 20 bis 80°C, vorzugsweise 30 bis 50°C, die Reaktionszeiten, je nach Reaktionstemperatur und Konzentration der Lauge etwa 1 bis 10, vorzugsweise 2 bis 4 Stunden. Bei Mitverwendung von PTK verringert sich die für die Umsetzung notwendige Menge an NaOH bzw. KOH um den Faktor 2 bis 10 im Vergleich zu den sonst benötigten Mengen.
   Das Verhältnis von organischer Phase zu wäßriger Phase sollte etwa 1:4 bis 10:1, vorzugsweise 1:1 bis 7:1 betragen.
   Da sich das Reaktionsgemisch bei Raumtemperatur nicht gut in 2 Phasen trennt, wird die Phasentrennung bei Temperaturen von etwa 40 bis 50°C, vorzugsweise gleich nach Abschluß der Umsetzung vorgenommen. Die abgetrennte organische Phase wird anschließend durch azeotrope Destillation entwässert. Eine derartige Hydrolysereaktion in einem Zweiphasensystem unter Verwendung von Phasentransferkatalysatoren wurde unseres Wissens noch nicht beschrieben.
c) Zur Durchführung der Vilsmeier-Chlorierung wird im allgemeinen zunächst in an sich bekannter Weise durch langsames Zufügen von SOCl₂ oder COCl₂ zu einem Gemisch aus Dimethylformamid (DMF) und dem erfindungsgemäßen Kohlenwasserstoff bei Temperaturen von 0 bis 20°C, vorzugsweise 3 bis 10°C der Vilsmeier-Komplex hergestellt. Hierzu wird dann bei Temperaturen von 0 bis 20°C, vorzugsweise etwa 3 bis 10°C im Verlauf von 0,5 bis 10 Stunden, vorzugsweise etwa 1 bis 3 Stunden die oben beschriebene rohe Lösung des Hydroxyacetals der allgemeinen Formel V zugefügt. Hierbei ist zu beachten, daß diese Lösung während der Zugabe gerührt werden sollte, da sie sich leicht entmischen kann. Nach einer Nachreaktion von 0,5 bis 10, vorzugsweise 1 bis 3 Stunden wird die Unterphase abgetrennt, mehrfach mit dem erfindungsgemäßen Kohlenwasserstoff extrahiert und die vereinigten organischen Phasen mit wäßriger Alkalilauge oder Wasser behandelt und dann mit verdünnter Alkalilauge oder Wasser gewaschen.
   Zur Erzielung optimaler Ausbeuten verwendet man für die erfindungsgemäße Vilsmeier-Chlorierung DMF und SOCl₂ oder COCl₂ im molaren Verhältnis 7:1 bis etwa 2:1. Die benötigte DMF-Menge beträgt im allgemeinen 2,0 bis 10, vorzugsweise 2,5 bis 4,0 Mol pro Mol des Hydroxyacetals der allgemeinen Formel V. Den Kohlenwasserstoff oder das Kohlenwasserstoffgemisch verwendet man mit Vorteil in Mengen von etwa 300 ml bis 1500 ml, vorzugsweise 500 bis 1100 ml Lösungsmittel pro Mol des Hydroxyacetals der Formel V.
   Die vereinigten organischen Extrakte werden mit vorzugsweise 10 %iger Alkalilauge und Wasser gewaschen.
   Von besonderer Bedeutung für das erfindungsgemäße Verfahren ist die überraschend vorteilhafte Durchführbarkeit der Umsetzung der 4-Chlor-acetale der Formel VI mit Triphenylphosphin, bei der gemäß dem Stand der Technik in Toluol nur Ausbeuten von 65 % der Theorie erhalten werden konnten. Diese Umsetzung kann erfindungsgemäß entweder in einem Alkanol mit 1 bis 3 C-Atomen oder in einem, vorzugsweise dem gleichen C₆- bis C₈-Kohlenwasserstoff oder -Kohlenwasserstoffgemisch wie die übrigen Reaktionsstufen durchgeführt werden. Aber auch Gemische aus einem Alkanol mit 1 bis 3 C-Atomen und einem C₆- bis C₈-Kohlenwasserstoff können vorteilhaft verwendet werden. Bei Anwesenheit eines Alkanols verringern sich die für eine vollständige Umsetzung notwendigen Reaktionszeiten drastisch.
d) Zur Durchführung der Umsetzung mit Triphenylphosphin wird aus der gemäß Stufe c) erhaltenen Lösung das Lösungsmittel ganz oder nur teilweise unter vermindertem Druck abdestilliert, der Rückstand in Methanol gelöst, die Lösung mit Triphenylphosphin versetzt und bis zu vollständiger Salzbildung unter Rückfluß zum Sieden erhitzt.
   Das C₁- bis C₃-Alkanol verwendet man hierbei in Mengen von 100 bis 1000 ml, vorzugsweise 250 bis 500 ml pro Mol 4-Chlor-acetal der Formel VI, das Triphenylphosphin in Mengen von etwa 1 bis 1,5 Mol pro Mol 4-Chlor-acetal der Formel VI. Die Reaktionszeit beträgt etwa 2 bis 8 Stunden. Auf diese Weise kann z.B. das 3-Formyl-2-butenyltriphenylphosphoniumchlorid mit 92 % Ausbeute aus 4-Chloracetal der Formel VI hergestellt werden.
   Es war überraschend, daß die Reaktionsstufe d) in niederen Alkanolen, insbesondere in Methanol, als Lösungsmittel so vorteilhaft verläuft, da man hätte erwarten können, daß das 4-Chlor-acetal der Formel VI durch das Alkanol zumindest teilweise in das 4-Alkoxy-acetal umgesetzt werden würde.

Zur Durchführung der Umsetzung mit Triphenylphosphin in einem C₆- bis C₈-Kohlenwasserstoff stellt man mit Vorteil die gemäß Stufe c) erhaltene Lösung des 4-Chloracetals der Formel VI auf eine Konzentration von etwa 10 bis 50 %, vorzugsweise 30 bis 40 %, ein und erhitzt zum Sieden. Anschließend versetzt man das Reaktionsgemisch mit dem Triphenylphosphin und erhitzt bis zu vollständiger Salzbildung unter Rückfluß zum Sieden. Triphenylphosphin verwendet man im allgemeinen in einer Menge von 1 Mol bis 1,5 Mol pro Mol 4-Chlor-acetal. Man kann aber auch die auf eine geeignete Konzentration eingestellte Lösung des 4-Chloracetals zunächst mit Triphenylphosphin versetzen und danach zum Sieden erhitzen. Die Reaktionszeiten betragen 6 bis 36 h, vorzugsweise 8 bis 24 h. Ausbeute: 95 % aus 4-Chloracetal VI.

Es war überraschend, daß durch Ersatz des gemäß dem Stand der Technik verwendeten Toluols als Lösungsmittel durch einen aliphatischen Kohlenwasserstoff die erzielbaren Ausbeuten wesentlich erhöht werden konnten.

Mit Hilfe des erfindungsgemäßen Verfahrens, insbesondere durch besondere Verfahrensführung bei der Überführung der 4-Acetoxyacetale der Formel IV in die 4-Hydroxyacetale der Formel V sowie bei der Umsetzung mit Triphenylphosphin können die für Terpensynthesen sehr begehrten cyclischen Acetale von 3-Formyl-2-butenyl-triphenylphosphoniumchlorid auch in technischem Maßstab auf relativ einfache Weise und mit außerordentlich guten Gesamtausbeuten hergestellt werden.

### Beispiel 1

a) 213,2 g (2,005 mol) Neopentylglykol wurden bei Raumtemperatur (RT) in 1,6 l n-Heptan suspendiert und mit 288,1 g (2 mol) (E)-3-Formyl-2-butenylacetat versetzt. Anschließend wurde im Reaktionsgefäß ein Unterdruck von ca. 200 mbar eingestellt, auf 40°C erwärmt und dann auf einmal eine Lösung von 0,76 g (4 mmol) p-Toluolsulfonsäure (Hydrat) in 0,76 g Wasser zugefügt. Die Temperatur des Reaktionsgemisches wurde auf 55 - 60°C erhöht und ein Azeotrop mit einem Siedepunkt von 40 bis 50°C abdestilliert. Nach etwa 35 Minuten (min) war das Reaktionsgemisch klar geworden, nach ca. 2 Stunden (h) hatten sich 36,5 ml Wasser abgeschieden.
b) In die auf 40°C abgekühlte Reaktionslösung von Stufe a) wurden 2 ml Protectol® KLC-50 (50 %ige Lösung in Wasser) addiert und 240 g (3 mol) einer 50 %igen wäßrigen NaOH; (δ = 1,52) im Verlauf von 1 h zugefügt. Anschließend wurde noch 2 h bei 40 bis 45°C gerührt, dann 180 ml Wasser zugefügt und nochmal 10 min gerührt. Bei 45 - 50°C trennten sich die beiden Phasen gut. Anschließend wurde die abgetrennte organische Phase durch azeotropes Abdestillieren bei 58°C/193 mbar entwässert.
c) 600 ml Dimethylformamid (DMF) und 600 ml n-Heptan wurden bei 5°C vorgelegt und innerhalb von 1 h bei 5 bis 10°C mit 297,4 g (2,5 mol) SOCl₂ versetzt, wobei der Vilsmeier-Komplex ausfiel. Anschließend wurde 30 min gerührt, dann innerhalb von 2 h bei 0 bis 5°C das gemäß Stufe b) erhaltene rohe (E)-3-(5,5-Dimethyl-1,3-dioxan-2-yl)-2-buten-1-ol durch einen gerührten Tropftrichter (wegen der Zweiphasigkeit in n-Heptan) zugefügt und noch 1 h bei 0 bis 5°C nachreagieren lassen. Zur Aufarbeitung trennte man die Unterphase ab, extrahierte diese 3x mit je 300 ml n-Heptan, rührte noch 15 min, wusch die vereinigten organischen Phasen mit einer Mischung aus 600 g einer 10%igen wäßrigen NaOH und 375 ml Wasser, rührte 15 min nach, trennte die Phasen und wusch die organische Phase bei 5 bis 10°C mit einer Mischung aus 110 g einer 50%igen wäßrigen NaOH und 440 ml Wasser. Zum Abschluß wusch man die organische Phase noch 3 mal mit je 300 ml Wasser.
   Die Ausbeute betrug 2290,6 g einer 14,8 gew.-%igen Lösung von (E)-2-(3-Chlor-1-methyl-1-propenyl)-5,5-dimethyl-1,3-dioxan in n-Heptan, entsprechend 339 g berechnet 100 % (1,656 mol) entsprechend einer Ausbeute von 82,8 % bezogen auf eingesetztes (E)-3-Formyl-2-butenylacetat(II).
d) Aus der gemäß Beispiel 1c) erhaltenen Lösung wurden bei 60°C und 100 mbar etwa 82 bis 87 % des Heptans abdestilliert, der Rückstand in 550 ml Methanol gelöst, mit 434,1 g (1,656 mol) Triphenylphosphin versetzt und für 2 h unter Rückfluß zum Sieden erhitzt.
   Die Ausbeute betrug 1218 g = 1221,7 ml einer Lösung, die 0,2 % Wasser und 67 % Feststoff (90 % Reinheit) enthielt. Gemäß Titration enthielt die Lösung 1,58 mol[(E)-3-(5,5-Dimethyl-1,3-dioxan-2-yl)-2-butenyl]-triphenylphosphoniumchlorid, entsprechend einer Ausbeute von 78,8 %, bezogen auf eingesetztes II. Die Verbindungen liegen als Isomerengemisch (ca. 10 Teile E-/1 Teil Z-Isomeres) vor.

### Beispiel 2

Aus der gemäß Beispiel 1c erhaltenen Lösung wurden bei 60°C/100 mbar etwa 50 % des Heptans abdestilliert. Diese aufkonzentrierte Lösung wurde mit 434,1 g (1,656 mol) Triphenylphosphin versetzt und für 16 h unter Rückfluß zum Sieden erhitzt. Nach Reaktionsende setzte man bei 30°C 500 ml Methanol zu, rührte gut auf und trennte die Phasen. Die methanolische Lösung (1160 g) enthielt zu 64 % das [(E)-3-(5,5-Dimethyl-1,3-dioxan-2-yl)-2-butenyl)-triphenylphosphoniumchlorid, entsprechend 1,59 mol = 79,6 % Ausbeute, bezogen auf eingesetztes 3-Formyl-2-butenylacetat.

### Beispiel 3

a) Acetalisierung von II mit Neopentylglykol in Heptan 319,7 g (3,01 mol) Neopentylglykol wurden bei RT in 2,5 l n-Heptan suspendiert und die Suspension mit 432 g (3 mol) (E)-3-Formyl-2-butenylacetat (99 %ig) versetzt. Anschließend wurde im Reaktionsgefäß ein Unterdruck von ca. 300 mbar eingestellt, auf 40°C erwärmt und dann auf einmal eine Lösung von 1,14 g (6 mmol) p-Toluolsulfonsäurehydrat in 1,14 g Wasser zugesetzt. Anschließend wurde das Reaktionsgemisch für 3 h auf Temperaturen von 53 bis 62°C bei 313 bis 277 mbar Druck erwärmt und dabei 53 ml Wasser ausgekreist. Gemäß gaschromatographischer Analyse betrug die Ausbeute an (E)-2-(3-Acetoxy-1-methyl-1-propenyl)-5,5-dimethyl-1,3-dioxan (IVa) 98,9 % der Theorie.
b) Verseifung von IVa ohne Phasentransferkatalysator (PTK) Die gemäß Beispiel 3a erhaltene rohe Lösung der Verbindung IVa wurde bei 40 - 50°C im Verlauf von 2 h unter Rühren mit insgesamt 280 ml (10,64 mol) einer 50%igen wäßrigen NaOH versetzt. Anschließend wurden 560 ml Wasser zugegeben, noch 15 min gerührt und nach 30 min Absetzen die Unterphase abgetrennt. Die Heptanphase wurde bei 40 - 50°C innerhalb von 15 min erneut mit 280 ml (10,64 mol) einer 50%igen wäßrigen NaOH versetzt, nach Zulaufende mit 560 ml Wasser versetzt, noch 15 min gerührt und nach 30 min Absetzen die Unterphase abgetrennt.
   Die Heptanphase wurde ohne Wasserwäsche bei Temperaturen von 46 - 51°C und 260 bis 200 mbar entwässert. Nach Einengen der Heptanphase erhielt man 542,3 g Rückstand enthaltend 85 Gew.-% (E)-2-(3-Hydroxy-1-methyl-1-propenyl)-5,5-dimethyl-1,3-dioxan (Va) entsprechend einer Ausbeute von 82,5 % der Theorie, bezogen auf eingesetzte Verbindung II.

### Beispiel 4

Verseifung von IVa mit PTK
115,75 g (0,5 mol) eines analog Beispiel 3a hergestellten (E)-2-(3-Acetoxy-1-methyl-1-propenyl)-5,5-dimethyl-1,3-dioxans (IVa) wurden bei RT in 400 ml n-Heptan gelöst, die Lösung auf 40°C erwärmt, mit 0,25 ml Aliquat® versetzt und im Verlauf von 5 min mit 60 g (0,75 mol) einer 50%igen wäßrigen NaOH versetzt. Anschließend wurde das Reaktionsgemisch noch etwa 2 h bei Temperaturen von etwa 45°C gerührt, wobei vorübergehend auch ohne Heizen und Kühlen Temperaturen von 55 - 60°C auftreten.

Zur Aufarbeitung versetzt man das Reaktionsgemisch unter kräftigem Rühren mit 90 ml Wasser und rührt etwa 5 min bei 44°C. Um zwei klare Phasen zu erhalten, wurde kurzzeitig auf 60°C erwärmt und bei dieser Temperatur die Phasentrennung vorgenommen. Nach Abdampfen des Heptans aus der warmen Heptanphase erhielt man 92,53 g an Va (99 %ig), entsprechend eine Ausbeute von 98,3 %, bezogen auf eingesetzte Verbindung IVa, bzw. 97,3 %, bezogen auf eingesetzte Verbindung II. Das cis/trans-Verhältnis der erhaltenen Verbindung Va betrug ca. 1:4.

### Beispiel 5

Vilsmeier-Chlorierung von Verbindung Va
285 ml (270,75 g = 3,7 mol) DMF und 750 ml n-Heptan wurden bei RT vorgelegt, auf 0°C gekühlt und innerhalb von 30 min bei 0 bis 5°C mit 138,2 g (1,15 mol) SOCl₂ versetzt, wobei der Vilsmeier-Komplex ausfiel. Anschließend rührte man noch 10 min nach und addierte dann innerhalb von 45 min bei 0 - 5°C eine gerührte Emulsion von 192 g (1 mol) der Verbindung Va (97 %ig) in 800 ml n-Heptan und ließ das Reaktionsgemisch noch 2 h bei dieser Temperatur nachreagieren.

Zur Aufarbeitung tropfte man in 1,5 h bei 10 bis 15°C 1420 g (3,55 mol) einer 10 %igen wäßrigen NaOH zu und rührte noch 5 min nach. Nach erfolgter Phasentrennung wurde die organische Phase mit 250 ml Wasser gewaschen. Man erhielt 1227,2 g einer 14,7 gew.-%igen Lösung von 2-(3-Chlor-1-methyl-1-propenyl)-5,5-dimethyl-1,3-dioxan (VIa) in Heptan, entsprechend einer Ausbeute von 88,1 % der Theorie, bezogen auf eingesetztes Va.

### Beispiel 6

285 ml DMF und 750 ml n-Heptan wurden bei RT vorgelegt, auf 0°C gekühlt und innerhalb von 30 min bei 0 bis 5°C mit 138,2 g SOCl₂ versetzt, das Reaktionsgemisch noch 10 min gerührt und dann innerhalb von 45 min bei 0 bis 5°C eine gerührte Emulsion aus 192 g (1 mol) der Verbindung Va (97%ig) in 800 ml n-Heptan zugefügt. Anschließend ließ man das Reaktionsgemisch noch 2 h bei dieser Temperatur reagieren (nach DC vollständiger Umsatz).

Zur Aufarbeitung wurde die Unterphase abgetrennt, noch 3x mit je 150 ml n-Heptan extrahiert, die vereinigten organischen Phasen auf 5°C gekühlt und dann innerhalb von 15 min mit einer Mischung aus 275 ml einer 10%igen wäßrigen NaOH und 190 ml Wasser versetzt. Nach 15 min bei 5 bis 10°C wurde die Unterphase abgetrennt und mit der organischen Phase die NaOH-Wäsche wiederholt. Zum Abschluß wurde die organische Phase noch 3x mit je 150 ml Wasser gewaschen.

Es verblieben 1877,4 g einer 10,41 gew.-%igen Lösung von 2-(3-Chlor-1-methyl-1-propenyl)-5,5-dimethyl-1,3-dioxan (VIa) in Heptan, entsprechend einer Ausbeute von 95,5 % der Theorie, bezogen auf eingesetzte Verbindung Va.

### Beispiel 7 (Umsetzung mit Phosgen)

a) 213,2 g (2,005 mol) Neopentylglykol wurden bei RT in 1,6 l n-Heptan suspendiert und mit 288,1 g (2 mol) (E)-3-Formyl-2-butenylacetat versetzt. Anschließend wurde im Reaktionsgefäß ein Unterdruck von ca. 200 mbar eingestellt, auf 40°C erwärmt und dann auf einmal eine Lösung von 0,47 g (5 mmol) Methansulfonsäure in 0,47 g Wasser zugefügt. Die Temperatur des Reaktionsgemisches wurde auf 60°C erhöht und ein Azeotrop mit einem Siedepunkt von 40 bis 50°C abdestilliert. Nach etwa 30 min war das Reaktionsgemisch klar geworden, nach ca. 2,5 h hatten sich 36 ml Wasser abgeschieden.
b) In die auf 40°C abgekühlte Reaktionslösung von Stufe a) wurden 2 ml Hexadecyltrimethylammoniumchlorid in Form einer 25 %igen Lösung in Wasser addiert und 240 g (3 mol) einer 50 %igen wäßrigen NaOH (δ = 1,52) im Verlauf von 1 h zugefügt. Anschließend wurde noch 2 h bei 40 bis 45°C gerührt, dann 180 ml Wasser zugefügt und nochmal 10 min gerührt. Bei 45 bis 50°C trennten sich die beiden Phasen gut. Anschließend wurde die abgetrennte organische Phase durch azeotropes Abdestillieren bei 58°C/200 mbar entwässert.
c) 500 ml DMF und 600 ml n-Heptan wurden bei 5°C vorgelegt und innerhalb von 1 h bei 5 bis 10°C mit 247,5 g (2,5 mol) eines auf -5°C gekühlten COCl₂ versetzt, wobei der Vilsmeier-Komplex ausfiel. Anschließend wurde 30 min gerührt, dann innerhalb von 2 h bei 0 bis 5°C das gemäß Stufe b) erhaltene rohe (E)-3-(5,5-Dimethyl-1,3-dioxan-2-yl)-2-buten-1-ol durch einen gerührten Tropftrichter zugefügt und noch 1 h bei 0 bis 5°C nachreagieren lassen. Zur Aufarbeitung trennte man die Unterphase ab, extrahierte diese 4x mit je 200 ml n-Heptan, rührte noch 15 min, wusch die vereinigten organischen Phasen mit einer Mischung aus 300 ml einer 5%igen wäßrigen NaOH und 375 ml Wasser, rührte 15 min nach, trennte die Phasen und wusch die organische Phase bei 5 bis 10°C 3 mal mit je 300 ml Wasser.
   Die Ausbeute betrug 2153 g einer 15,8 gew.-%igen Lösung von (E)-2-(3-Chlor-1-methyl-1-propenyl)-5,5-dimethyl-1,3-dioxan in n-Heptan, entsprechend 340 g berechnet 100 % (1,66 mol) entsprechend einer Ausbeute von 83 % bezogen auf eingesetztes (E)-3-Formyl-2-butenylacetat(II).
d) Aus der gemäß Stufe c) erhaltenen Lösung wurden bei 60°C und 100 mbar etwa 50 % des Heptans abdestilliert, der Rückstand mit 435 g (1,66 mol) Triphenylphosphin versetzt und für 20 h unter Rückfluß zum Sieden erhitzt. Nach Abkühlen auf Raumtemperatur gab man 600 ml MeOH zu, rührte gut auf, trennte die Phasen und erhielt 1293 g einer methanolischen Lösung, die 0,2 % Wasser und 62 % Feststoff (95 % Reinheit) enthielt. Gemäß Titration enthielt die Lösung 1,6 mol [(E)-3-(5,5-Dimethyl-1,3-dioxan-2-yl)-2-butenyl]-triphenylphosphoniumchlorid, entsprechend einer Ausbeute von 80 %, bezogen auf eingesetztes 3-Formyl-2-butenylacetat. Die Verbindungen liegen als Isomerengemisch (ca. 10 Teile E-/1 Teil Z-Isomers) vor.

### Beispiel 8

Aus einer gemäß Beispiel 7a bis 7c erhaltenen Lösung wurden bei 60°C und 100 mbar etwa 50 % des Heptans abdestilliert, der Rückstand mit 435 g (1,66 mol) Triphenylphosphin und 600 ml Methanol versetzt und für 2 h unter Rückfluß zum Sieden erhitzt. Nach Abkühlen auf RT trennte man die gebildeten Phasen und erhielt 1290 g einer methanolischen Lösung, die 0,2 % Wasser und 62 % Feststoff (95 % Reinheit) enthielt. Gemäß Titration enthielt die Lösung 1,6 Mol [(E)-3-(5,5-Dimethyl-1,3-dioxan-2-yl)-2-butenyl]-triphenylphosphoniumchlorid, entsprechend einer Ausbeute von 80 %, bezogen auf eingesetztes 3-Formyl-2-butenylacetat. Die Verbindungen liegen als Isomerengemisch (ca. 10 Teile E-/1 Teil Z-Isomeres) vor.

### Beispiel 9

Aus einer gemäß Beispiel 1c erhaltenen Lösung wurden bei 60°C/100 mbar etwa 70 % des Heptans abdestilliert. Diese aufkonzentrierte Lösung wurde mit 434,1 g (1,656 mol) Triphenylphosphin und 600 ml Ethanol versetzt und für 2,5 h unter Rückfluß zum Sieden erhitzt. Nach Reaktionsende trennte man bei 30°C die Phasen. Die ethanolische Lösung (1250 g) enthielt zu 59 % das [(E)-3-(5,5-Dimethyl-1,3-dioxan-2-yl)-2-butenyl]-triphenylphosphoniumchlorid, entsprechend 1,58 Mol = 79 % Ausbeute, bezogen auf eingesetztes 3-Formyl-2-butenylacetat.

## Patentansprüche

1. Verbessertes Verfahren zur Herstellung von cyclischen Acetalen des 3-Formyl-2-butenyl-triphenylphosphoniumchlorids der allgemeinen Formel I 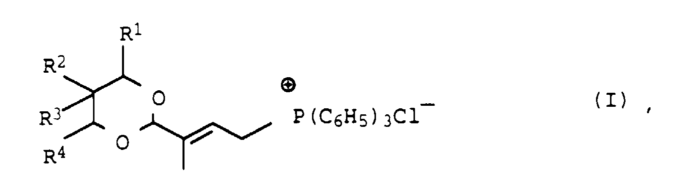 in denen
R¹, R², R³ oder R⁴ für H oder -CH₃ stehen, durch
a) Acetalisieren das 3-Formyl-butenylacetates der Formel II 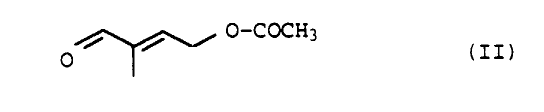 mit 1,3-Diolen der allgemeinen Formel III 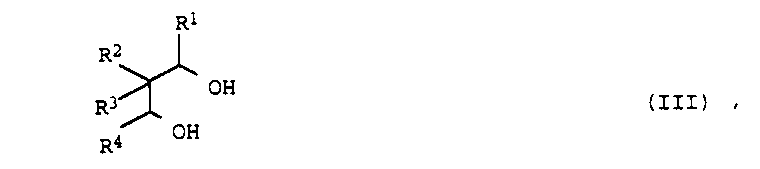 in der R¹ - R⁴ die oben angegebene Bedeutung haben,
b) Überführen der erhaltenen Acetoxyacetale der allgemeinen Formel IV in die entsprechenden Hydroxyacetale der allgemeinen Formel V 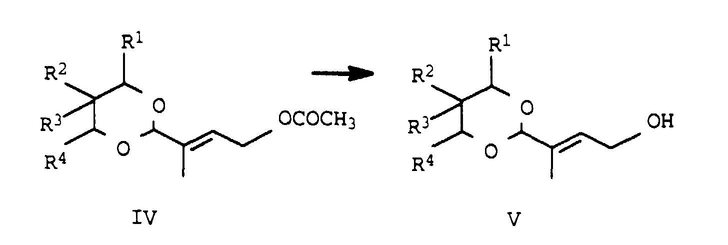
c) Vilsmeier-Chlorierung der erhaltenen Hydroxyacetale der Formel V unter Bildung der 4-Chlor-acetale der allgemeinen Formel VI 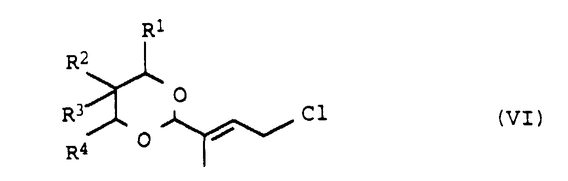 und
d) Umsetzung der 4-Chlor-acetale der Formel VI mit Triphenylphosphin,
das dadurch gekennzeichnet ist, daß man die Reaktionsschritte a) bis c) in einem aliphatischen oder cycloaliphatischen Kohlenwasserstoff oder Kohlenwasserstoffgemisch mit 6 bis 8 C-Atomen und den Reaktionsschritt d) in einem Alkanol mit 1 bis 3 C-Atomen und/oder in einem aliphatischen oder cycloaliphatischen Kohlenwasserstoff bzw. Kohlenwasserstoffgemisch mit 6 bis 8 C-Atomen durchführt.

2. Verfahren gemäß Anspruch 1, dadurch gekennzeichnet, daß man die Reaktionsschritte a) bis c) oder a) bis d) in dem gleichen Kohlenwasserstoff oder Kohlenwasserstoffgemisch durchführt.

3. Verfahren gemäß Anspruch 1, dadurch gekennzeichnet, daß man das Überführen der Acetoxyacetale der allgemeinen Formel IV in die entsprechenden Hydroxyacetale der allgemeinen Formel V gemäß Verfahrensschritt b) mit mindestens 5 %iger wäßriger Natronlauge oder Kalilauge durchführt.

4. Verfahren gemäß Anspruch 3, dadurch gekennzeichnet, daß man das Überführen der Acetoxyacetale der allgemeinen Formel IV in die entsprechenden Hydroxyacetale der allgemeinen Formel V gemäß Verfahrensschritt b) mit wäßriger Natronlauge oder Kalilauge in Gegenwart von Phasentransferkatalysatoren durchführt.

5. Verfahren gemäß Anspruch 1, dadurch gekennzeichnet, daß man im Reaktionsschritt d) die 4-Chloracetale der allgemeinen Formel VI in einem Gemisch aus Methanol und einem aliphatischen oder cycloaliphatischen Kohlenwasserstoff oder Kohlenwasserstoffgemisch mit 6 bis 8 C-Atomen als Lösungsmittel mit Triphenylphosphin umsetzt.

6. Verbessertes Verfahren zur Herstellung von cyclischen Acetalen des 3-Formyl-2-butenyl-triphenylphosphoniumchlorids der allgemeinen Formel I 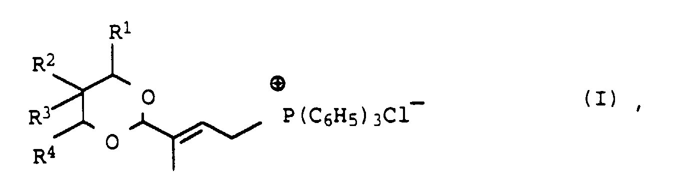 in denen
R¹, R², R³ oder R⁴ für H oder -CH₃ stehen, dadurch gekennzeichnet, daß man die 4-Chlor-acetale der allgemeinen Formel VI 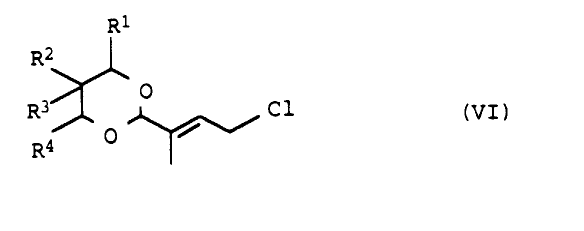 in einem Alkanol mit 1 bis 3 C-Atomen und/oder einem aliphatischen oder cycloaliphatischen Kohlenwasserstoff mit 6 bis 8 C-Atomen als Lösungsmittel mit Triphenylphosphin umsetzt.

## Claims

1. An improved process for preparing cyclic acetals of 3-formyl-2-butenyltriphenylphosphonium chloride of the formula I 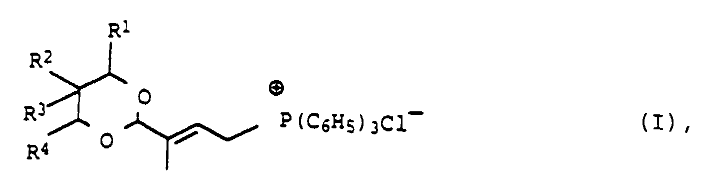 where R¹, R², R³ and R⁴ are each H or CH₃, by
a) acetalizing the 3-formylbutenyl acetate of the formula II 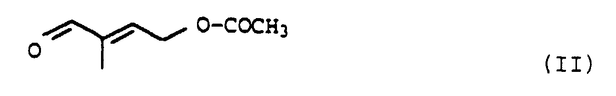 with 1,3-diols of the formula III 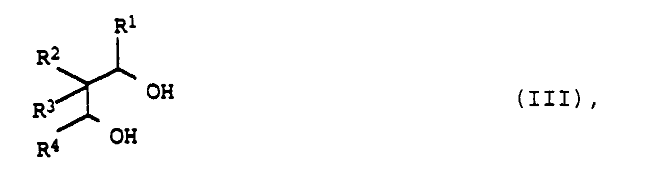 where R¹-R⁴ have the abovementioned meanings,
b) converting the resulting acetoxy acetals of the formula IV into the corresponding hydroxy acetals of the formula V 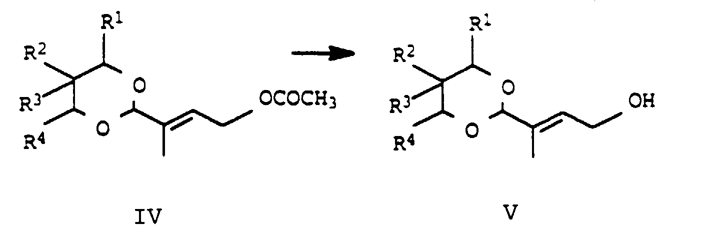
c) subjecting the resulting hydroxy acetals of the formula V to Vilsmeier chlorination to form the 4-chloro acetals of the formula VI 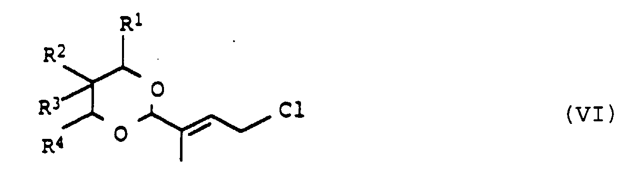 and
d) reacting the 4-chloro acetals of the formula VI with triphenylphosphine,
wherein steps a) to c) are carried out in an aliphatic or cycloaliphatic hydrocarbon or mixture of hydrocarbons with 6-8 carbons, and step d) is carried out in an alkanol with 1-3 carbons and/or in an aliphatic or cycloaliphatic hydrocarbon or mixture of hydrocarbons with 6-8 carbons.

2. A process as claimed in claim 1, wherein steps a) to c) or a) to d) are carried out in the same hydrocarbon or mixture of hydrocarbons.

3. A process as claimed in claim 1, wherein the conversion of the acetoxy acetals of the formula IV into the corresponding hydroxy acetals of the formula V in step b) is carried out with at least 5% strength aqueous sodium hydroxide or potassium hydroxide solution.

4. A process as claimed in claim 3, wherein the conversion of the acetoxy acetals of the formula IV into the corresponding hydroxy acetals of the formula V in step b) is carried out with aqueous sodium hydroxide or potassium hydroxide solution in the presence of phase-transfer catalysts.

5. A process as claimed in claim 1, wherein the reaction in step d) of the 4-chloro acetals of the formula VI with triphenylphosphine is carried out in a mixture of methanol and an aliphatic or cycloaliphatic hydrocarbon or mixture of hydrocarbons with 6-8 carbons as solvent.

6. An improved process for preparing cyclic acetals of 3-formyl-2-butenyltriphenylphosphonium chloride of the formula I 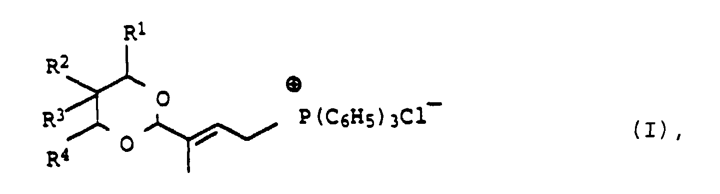 where R¹, R², R³ and R⁴ are each H or CH₃, which comprises reacting the 4-chloro acetals of the formula VI 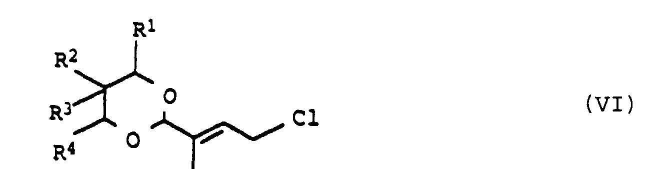 with triphenylphosphine in an alkanol with 1-3 carbons and/or an aliphatic or cycloaliphatic hydrocarbon with 6-8 carbons as solvent.

## Revendications

1. Procédé perfectionné pour préparer des acétals cycliques du chlorure de 3-formyl-2-butényl-triphénylphosphonium répondant à la formule générale I 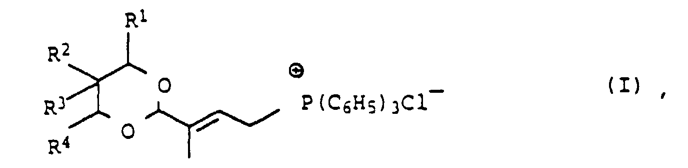 dans laquelle
R¹, R², R³ ou R⁴ représentent chacun H ou -CH₃, par
a) acétalisation de l'acétate de 3-formyl-butényle de formule II 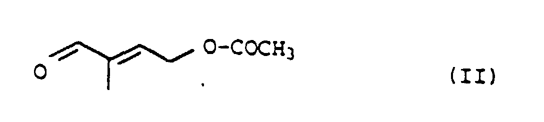 à l'aide de 1,3-diols de formule générale III 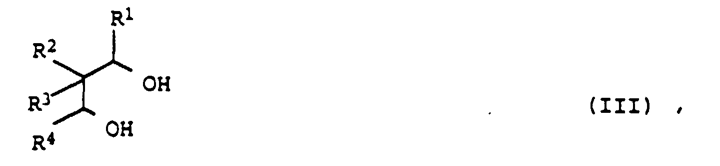 dans laquelle R¹ à R⁴ ont les significations indiquées ci-dessus,
b) conversion des acétoxyacétals obtenus, répondant à la formule générale IV, en les hydroxyacétals correspondants de formule générale V 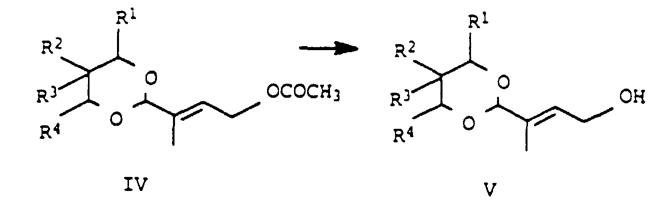
c) chloruration selon Vilsmeier des hydroxyacétals obtenus, répondant à la formule V, avec formation des 4-chloro-acétals de formule générale VI 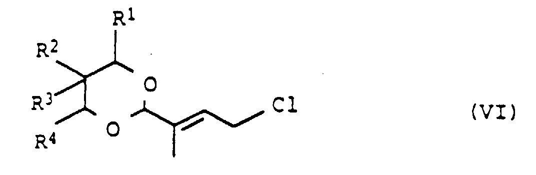 et
d) réaction des 4-chloro-acétals de formule VI avec la triphénylphosphine, caractérisé par le fait que les stades de réaction a) à c) sont réalisés dans un hydrocarbure ou un mélange d'hydrocarbures aliphatiques ou cycloaliphatiques en C6-C8 et le stade de réaction b) dans un alcool en C1-C3 et/ou dans un hydrocarbure aliphatique ou cycloaliphatique ou un mélange de tels carbures en C6-C8.

2. Procédé selon la revendication 1, caractérisé par le fait que l'on réalise les stades de réaction a) à c) ou a) à d) dans le même hydrocarbure ou mélange d'hydrocarbures.

3. Procédé selon la revendication 1, caractérisé par le fait que, dans le stade opératoire b), on convertit les acétoxyacétals de formule générale IV en les hydroacétals correspondants de formule générale V à l'aide d'une lessive de soude ou de potasse à une concentration d'au moins 5 %.

4. Procédé selon la revendication 3, caractérisé par le fait que, au stade opératoire b), on convertit les acétoxyacétals de formule générale IV en les hydroacétals correspondants de formule générale V à l'aide d'une lessive de soude ou de potasse en présence de catalyseurs à transfert de phase.

5. Procédé selon la revendication 1, caractérisé par le fait que, au stade de réaction d), on fait réagir les 4-chloroacétals de formule générale VI avec la triphénylphosphine dans un solvant consistant en un mélange de méthanol et d'un hydrocarbure ou mélange d'hydrocarbures aliphatiques ou cycloaliphatiques en C6-C8.

6. Procédé perfectionné pour préparer les acétals cycliques du chlorure de 3-formyl-2-butényl-triphénylphosphonium de formule générale I 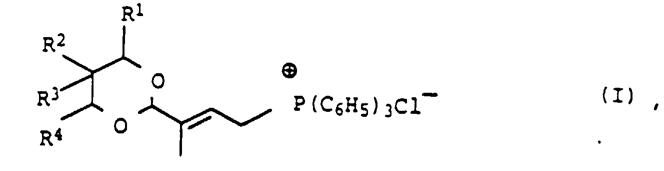 dans laquelle
R¹, R², R³ ou R⁴ représentent chacun H ou -CH₃, caractérisé par le fait que l'on fait réagir les 4-chloro-acétals de formule générale VI 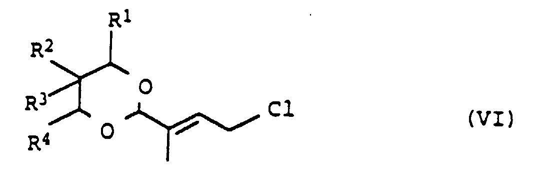 avec la triphénylphosphine dans un alcanol en C1-C3 et/ou un hydrocarbure aliphatique ou cycloaliphatique en C6-C8 servant de solvant.
